# EUROPEAN PATENT APPLICATION

(11) **EP 2 754 649 A1**
(43) Date of publication of application: **16.07.2014**
(21) Application number: 13000112.6
(22) Date of filing: 10.01.2013
(51) Int. Cl.: C07C 51/265, G01N 30/06

(54) **Method for determinng impurity concentration in terephthalic acid**

(71) Applicant: Saudi Basic Industries Corporation, Riyadh 11422 (SA)
(72) Inventor: The designation of the inventor has not yet been filed
(74) Representative: Vroemen, Maurice

(57) **Abstract**

The present invention relates to a method for determining the concentration of 4-carboxy benzaldehyde in a terephthalic acid composition, comprising a) reacting 4-carboxy benzaldehyde in the terephthalic acid composition with 2,4-dinitrophenylhydrazine to obtain 4-carboxy benzaldehyde 2,4-dinitrophenylhydrazone and b) determining the concentration of 4-carboxy benzaldehyde 2,4-dinitrophenylhydrazone.

## Description

The present invention relates to a method for determining the concentration of impurities in a terephthalic acid composition. In particular, the invention relates to a method for determining the concentration of 4-carboxy benzaldehyde (4-CBA) in a terephthalic acid composition.

Terephthalic acid (TPA) and isopthalic acid (IPA), also known as 1,4- and 1,3-benzenedicarboxylic acid, are produced on large scale as key raw materials for various polymers; including thermoplastics like PET and PBT, and thermosetting polyester resins. Most of the commercially applied processes for making aromatic polycarboxylic acids like TPA are based on the technology originally described by US 2833816. A soluble cobalt-manganese-bromine catalyst system forms the heart of these processes, yielding nearly quantitative oxidation of the starting xylene methyl groups. Acetic acid is generally used as solvent, and oxygen in compressed air is the oxidant, with reaction temperature in the range of 170-220 degrees centigrade. Various salts of cobalt and manganese can be used as catalyst, and the bromine source can be, amongst others, HBr, NaBr, ammoniumbromide or tetrabromoethane. The bromide is considered to act as a promoter, especially needed for activating the oxidation of the second (and further) methyl substituent. The oxidation reaction is performed in the liquid phase, but owing to the low solubility of the aromatic polycarboxylic acid in the solvent, most of it precipitates as it forms. This yields in fact a three-phase system: solid TPA crystals; solvent with dissolved starting aromatic compound, intermediate products, and some dissolved TPA; and vapour comprising nitrogen, acetic acid, water, and oxygen. The heat of reaction is removed by solvent evaporation. Typically, residence times of up to about 120 min. are used, resulting in over 98 percent of xylene being reacted and yield to TPA of over 95 percent.

The oxidation of the methyl groups occurs in steps, with e.g. in case of p-xylene two intermediates being formed, i.e. p-toluic acid and 4-carboxybenzaldehyde (4-CBA; also called 4-formylbenzoic acid). 4-CBA is a troublesome compound, owing to its structural similarity to TPA, resulting in co-crystallisation with and in entrapment in the TPA. For making high molar mass linear polyester, monomers like terephthalic acid need to be of very high purity.

Although the initial product obtained with a process as described above may contain greater than 98 percent TPA, it is generally referred to as crude terephthalic acid (CTA). In order to make polymer grade TPA, commonly referred to as purified terephthalic acid (PTA), processes generally further comprise one or more reaction, purification and/or crystallisation steps.

The processing steps for purifying CTA into PTA are well known to the skilled person, and have been described in general literature, for example in relevant chapters of Ullmann's Encyclopedia of Industrial Chemistry (e.g. J. Sheehan, R. 2000. Terephthalic Acid, Dimethyl Terephthalate, and Isophthalic Acid. Ullmann's Encyclopedia of Industrial Chemistry), and in above-cited patent publications, and literature references cited therein. Such further processing steps may include isolation steps like filtration or centrifugation, washing steps, secondary reaction steps like hydrogenation or post-oxidation, and recrystallisation and drying steps.

Generally the level of 4-CBA in PTA is used as an indication of the total level of impurities present in the sample during the manufacturing process. Presence of impurities in terephthalic acid slows down the polymerization process and also imparts undesirable coloration to the polymer. It is thus important to know the concentration of 4-CBA in a PTA composition.

Some methods are known for determining the concentration of 4-CBA in a CTA or PTA composition. Lourdes et.al, J. Braz. Chem. Soc., Vol. 15, No. 3, 400-406, 2004 describes a method for determining the concentration of 4-CBA along with other impurities in a CTA or PTA composition using capillary electrophoresis method. US5166420 relates to the production of high purity TA from a crude TA. The content of 4-CBA contained in the crude terephthalic acid was determined by means of polarographic analysis. Polarographic determination of 4-carboxybenzaldehyde in terephthalic acid, Metrohm Technical document AB 190/2 e uses polarography. These methods require expensive equipments which are not commonly available.

A fluorescence method for determining the levels of 4-CBA in CTA developed by James Daniels et al, Polymer Degradation and Stability 65 (1999) 347-353 is available. However, the fluorescence properties of 4-CBA in solution are dependent on the polar and acidic properties of the solvent used and method is time consuming.

Chromatographic method using UV detection described in Pradip Munshi et.al., Anal. Methods, 2010, 2, 382-386 is useful for analysis of 4-CBA in reaction mixtures. However, it was found that the method is not very accurate when analysing the concentration of 4-CBA in PTA powder.

It is an object of the present invention to provide a rapid, simple, quantitative method for determination of the concentration of (4-CBA) in a PTA composition.

Accordingly, the present invention provides a method for determining the concentration of 4-carboxy benzaldehyde (4-CBA) in a terephthalic acid (TA) composition, comprising
a) reacting 4-CBA in the TA composition with 2,4-dinitrophenylhydrazine (DNPH) to obtain 4-carboxy benzaldehyde 2,4-dinitrophenylhydrazone (4-CBA DNPH) and
b) determining the concentration of 4-CBA DNPH.

It was found that the concentration of 4-CBA DNPH can be determined with a higher accuracy than the concentration of 4-CBA. Although not wishing to be bound by any theory, the inventors assume that 4-CBA complexes with TA making the direct analysis difficult. This problem was overcome by producing 4-CBA DNPH which does not complex with TA. The concentration of 4-CBA DNPH determined by this method was found to accurately correspond to the concentration of 4-CBA. Accordingly, the concentration of 4-CBA can be calculated from the measured concentration of 4-CBA DNPH.

Step b) may be performed by any known methods, but preferably high performance liquid chromatography (HPLC) is used. HPLC is advantageous in that it is commonly available. Reverse phase high performance liquid chromatography is especially preferred. Preferably, the HPLC involves detecting the concentration of 4-CBA DNPH by a ultraviolet (UV) detector, preferably at 385 nm wavelength.

The TA composition especially useful for use with the method according to the invention is used is a TA composition made by step-wise oxidation of p-xylene. The term "step-wise oxidation of p-xylene" is understood to mean a series of reaction as follows:

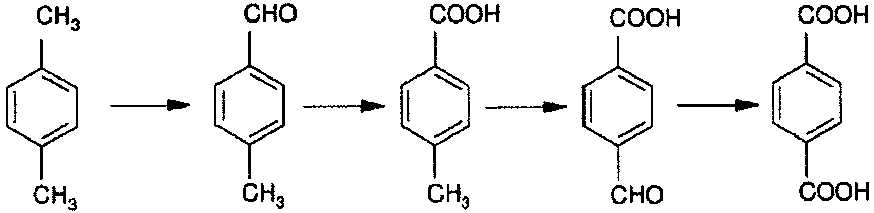

The TA composition for which the method according to the invention is used is preferably a purified TA composition. Preferably, the concentration of 4-CBA in the TA composition is at most 357 µg/g, as determined by the method according to the invention. Preferably, the concentration of 4-CBA in the TA composition is at least 5.8 µg/g, as determined by the method according to the invention. It was found that the determination of the concentration in this range of 4-CBA was particularly accurate.

The TA composition subjected to step a) may be a liquid reaction product from the step-wise oxidation of p-xylene. Alternatively, the TA composition subjected to step a) may also be a powder composition which has undergone a purification process. The powder composition may be dissolved in dimethyl sulfoxide (DMSO). The ratio between DMSO and the powder composition may e.g. be 10-30mL, preferably 15-25 mL per g of the TA composition.

A HCl solution of DNPH may be added to the DMSO solution. The HCl solution preferably has a DNPH concentration of 0.01-0.1 M or 0.03-0.08 M or about 0.05 M. The amount of DNPH to be added may e.g. be 0.01 to 0.1 mmol per g of the powder composition, preferably 0.04-0.06 mmol per g of the TA composition.

In a particularly preferred embodiment, the following amounts of compounds are used per g of the powder composition:
10 mL of DMSO to make a DMSO solution of the TA composition
1.0 mL of HCl solution of 0.05 M of DNPH

A further aspect of the present invention provides a process for setting a condition for purifying a crude TA composition. The condition may be any parameter which may influence the purity of the TA composition and include temperature, pressure, compounds used in the purification process. Accordingly, the process for setting a condition for purifying a crude TA composition comprises the steps of: purifying the crude TA composition at multiple conditions, determining for each condition the concentration of 4-CBA in the purified TA composition by the method according to the invention and setting the condition based on the determined concentrations of 4-CBA for the respective condition. Usually, the condition which resulted in the lowest 4-CBA concentration is set as the condition for purifying the crude TA composition.

A further aspect of the present invention provides a process for the preparation of a purified TA composition. The process comprises: performing the process for setting a condition for purifying a crude TA composition and purifying a crude TA composition according to the condition set by the process for setting the condition.

Although the invention has been described in detail for purposes of illustration, it is understood that such detail is solely for that purpose and variations can be made therein by those skilled in the art without departing from the spirit and scope of the invention as defined in the claims.

It is further noted that the invention relates to all possible combinations of features described herein, preferred in particular are those combinations of features that are present in the claims.

It is further noted that the term 'comprising' does not exclude the presence of other elements. However, it is also to be understood that a description on a product comprising certain components also discloses a product consisting of these components. Similarly, it is also to be understood that a description on a process comprising certain steps also discloses a process consisting of these steps.

The invention will now be further illustrated with below described experiments.

### Experiments

### Comparative experiment

Trials were carried out by referring and following the chromatographic methods available in Pradip Munshi et.al., Anal. Methods, 2010, 2, 382-386. However, the suggested methodology of direct analysis of the concentration of 4-CBA in PTA by injecting supernatant liquid into HPLC did not yield the suitable results and lacked accuracy.

### General procedure of the experiments of the method according to the invention

### 1-1. Calibration graph and its linearity

Known amounts of 4-CBA were dissolved in DMSO to prepare solutions having known concentrations of 4-CBA. These solutions were mixed with a HCl solution of DNPH. The solutions were injected into HPLC and the responses were measured. A good linear correspondence was observed between the concentration of 4-CBA and the response of the HPLC based on 4-CBA DNPH. A calibration graph indicating the relationship between the concentration of 4-CBA and the response of the HPLC based on 4-CBA DNPH was obtained.

### 1-2. Matrix effect

The presence of matrix effect of DNPH on 4-CBA was analyzed. A PTA sample dissolved in DMSO spiked with a known amount of 4-CBA was analyzed and the result was checked against the calibration graph obtained as described above. The degree of agreement between the real amount added and the amount determined according to the calibration graph was well within the acceptable limit. This showed the method had sufficient accuracy for the analysis of 4-CBA.

Hence, it was confirmed that the 4-CBA concentration can be determined by measuring the concentration of 4-CBA DNPH.

1-3. Measurement of 4-CBA concentration in PTA powder PTA powder with unknown concentration of 4-CBA were dissolved in DMSO. The solution was mixed with a HCl solution of DNPH. The solution was injected into HPLC and the response was compared against the calibration graph, to determine the concentration of 4-CBA in the PTA powder.

### 1-4. Optimization of the method

To study the effect of the amount of DNPH required to complete the derivatization (formation of 4-CBA DNPH), different amounts of DNPH was added to a solution with a known concentration of 4-CBA.

Additionally, the following experiments were performed to verify the method according to the invention.

### 1-5. Precision (repeatability)

The precision of the method in terms of retention time and peak area of 4-CBA DNPH was determined by conducting repeated injections of a 4-CBA DNPH solution with the same known concentrations. The results indicated good precision of the method.

### 1-6. Specificity to 4-CBA DNPH

The specificity of the method was determined by analyzing a sample solution prepared without the addition of the PTA composition. No interfering peak was observed at the retention time of the analyte. This result indicates that the method is specific for the analysis of 4-CBA DNPH.

### 1-7. Quantification limit and Detection limit

The quantification limit and the detection limit were determined by injecting low concentrations of 4-CBA standard and evaluating the signal/noise (S/N) ratios.

### Specific procedure of the experiments of the method according to the invention

### Reagents, chemicals and standards

Acetonitrile was HPLC grade purchased from Merck (Mumbai, India) and DMSO was AR grade purchased from Chemlabs (Leonid Chemicals, Bangalore), which were used as solvents. Standard p-carboxybenzaldehyde (4-CBA) was purchased from Aldrich (Taiwan) and pure grade 2,4 dinitrophenylhydrazine (DNPH) was purchased from Merck (Mumbai, India). Water was purified by Milli-Q water purification system (Millipore, France). Acetic acid glacial AR grade and hydrochloric acid (HCl) LR grade was purchased from Chemlabs (Leonid Chemicals, Bangalore). Purified terephthalic acid samples manufactured by different producers were provided by SABIC, Riyadh research facility.

### Instrumentation

The experiments were conducted in Agilent HPLC system (model 1100, USA), equipped with diode array detector. Samples were introduced by auto injection. Waters LCMS system (model Micromass, Quattro Ultima, UK) was used to characterize the 4-CBA hydrazone derivative.

### Chromatographic condition

The separation was obtained in Agilent (150 mm x 4.6 mm x 5 µm) Eclipse Zorbax C18 column (USA). A mixture of acetonitrile and 1.0% aqueous acetic acid solution (w/v) in a ratio of 1:1 v/v was used as the mobile phase in isocratic mode. Optimum separation was achieved at flow rate 1.0 mL min⁻¹. The detection wavelength was fixed at 385 nm. The developed LC method was validated with respect to specificity, linearity, accuracy and precision. Column oven was kept at ambient temperature (∼25°C). The chromatographic run time was set at 15 min. The column was pre-conditioned for 30 min with mobile phase before starting the analysis.

### 2-1. Calibration graph and its linearity

Stock solutions of 4-CBA in DMSO and 2, 4-DNPH in dilute hydrochloric acid were prepared as follows:
The stock solution of 4-CBA standard (1000 µg/mL) was prepared by dissolving appropriate amounts in the DMSO. A working solution having a concentration of 100µg/mL and 10µg/mL was prepared from above stock solution in DMSO.
The stock solution of 2,4-DNPH in HCl was prepared by weighing 100mg of DNPH reagent in 10 mL volumetric flask. 3mL of concentrated hydrochloric acid was added to the flask. The flask was sonicated for 15 minutes and the volume was adjusted to 10mL with water. This solution was thoroughly mixed and filtered using 0.45µm Teflon filter. The concentration of the obtained solution was about 0.05M DNPH.

Calibration standards were made from the above stock solutions. 5 mL DMSO was added into different 10 mL volumetric flasks. Further 0.4 mL of the DNPH solution was added to the volumetric flask. Different amounts of above prepared stock solution of 4-CBA was added to each of the above 10 mL volumetric flasks. The mixture was hand shaken for ∼3 min. In each case, the volume was made up to the mark using DMSO and kept aside for 15 minutes. After 15 min, 0.5mL aliquots were transferred into separate 10 mL volumetric flasks, and were then diluted to 10 mL with DMSO.

Each solution was injected into HPLC in duplicate and the mean of 4-CBA hydrazone peak area was considered. The response was measured at 385nm using the UV detector. Peak area obtained was plotted against respective concentrations and least-squares linear regression (R²) analysis was performed, as shown in Table 1.

**Table 1**

| Method linearity data | | | | | |
|---|---|---|---|---|---|
| Compound | Slope | Intercept | r2 | range (µg/mL) | n |
| 4-CBA | 356.2569 | 2.1206 | 1.0000 | 0.025 -1.50 | 6 |

A plot of concentration versus mean area shows a linear relationship with regression (R²) of 1.0000 in the range of 0.025 µg/mL to 1.50 µg/mL

### 2-2. Matrix effect

The method was validated for its accuracy by carrying out an experiment to verify the presence of matrix effect. In this case, the PTA sample was dissolved in DMSO spiked with known amount of 4-CBA, derivatized and analyzed. The relative area increment was interpolated in the calibration graph obtained as described above. The result of the experiment is shown in Table 2. The degree of agreement between the real amount added and that determined by interpolation is well within the acceptable limit. Recovery obtained was in the range of 99-107%. This showed the method had sufficient accuracy for the analysis of 4-CBA.

**Table 2.**

| Accuracy data: | | |
|---|---|---|
| Analyte | µg(added) | µg (calculated) |
| 4-CBA | 5.95 | 6.17 |

2-3 Measurement of 4-CBA concentration in PTA powder Eight PTA compositions (0.5 g) from different manufacturers with unknown amounts of 4-CBA were weighed and placed in a 10 mL volumetric flask. 5mL of DMSO was added. The PTA composition was dissolved by sonication for 15 minutes. 0.4mL of the 0.05 M 2, 4-DNPH solution as obtained above was added. The mixture was hand shaken for ∼3min. In each case, the volume was made up to the mark (10 mL) using DMSO and kept aside for 15 minutes. After 15 min, 0.5mL aliquots were transferred into 10 mL volumetric flasks, and were then diluted to the mark with DMSO and injected into HPLC in duplicate. The response was measured at 385nm against an appropriately prepared blank without PTA. Sample preparation was carried out in triplicate.

Using the calibration graph obtained as described in 2-1, the concentrations of 4-CBA in each of the PTA composition were determined, as shown in Table 3.

**Table 3**

| Concentration of 4-CBA in 7 different PTA samples determined by HPLC analysis after derivatisation with 2, 4 DNPH^{a}. | | | |
|---|---|---|---|
| Sample | 4-CBA content (µg/g) | SD | %RSD |
| 1 | 13.22 | 0.222 | 1.68 |
| 2 | 13.52 | 0.240 | 1.78 |
| 3 | 13.55 | 0.084 | 0.62 |
| 4 | 13.20 | 0.066 | 0.50 |
| 5 | 19.62 | 0.258 | 1.31 |
| 6 | 144.49 | 3.662 | 2.53 |
| 7 | 246.21 | 2.156 | 0.88 |

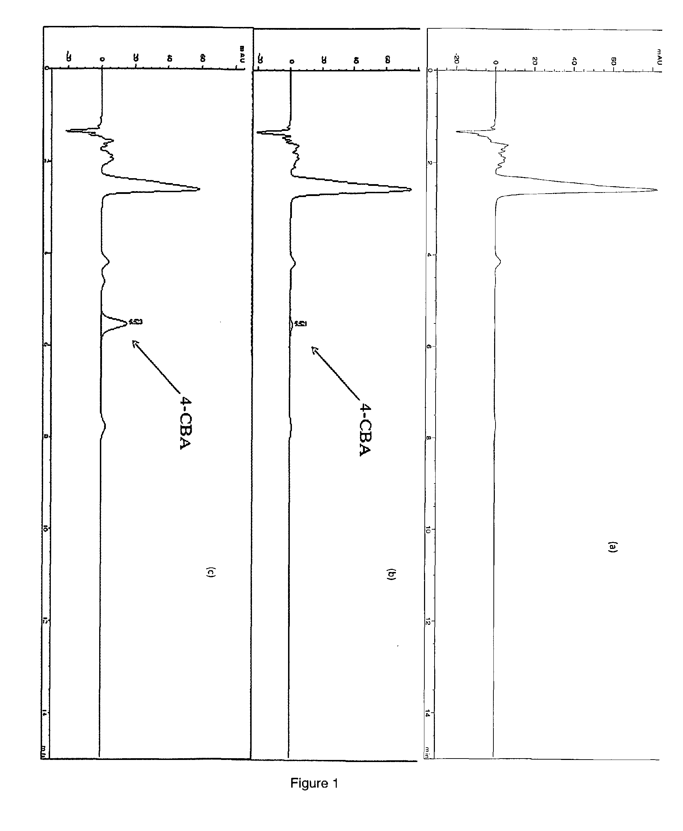

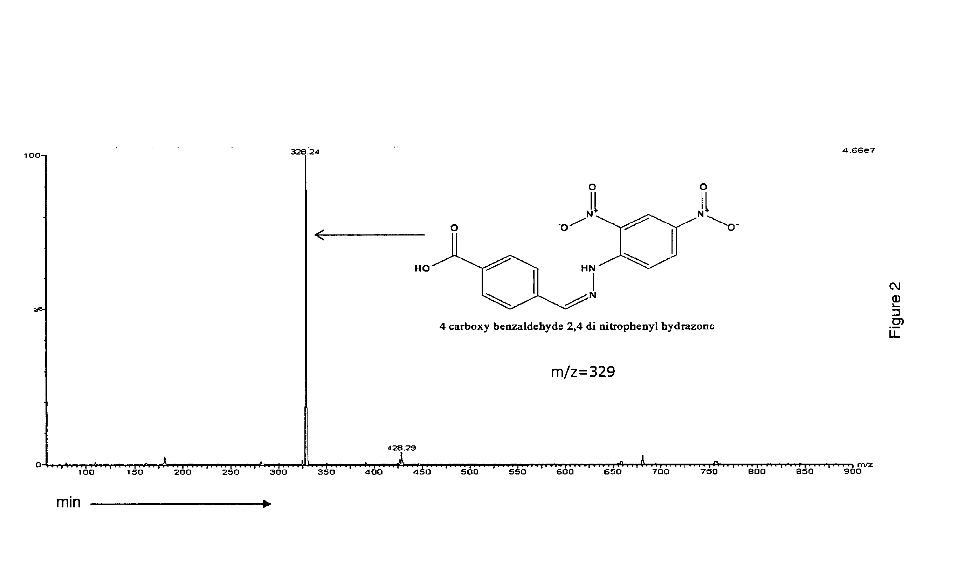

A typical chromatogram of the sample can be seen in Fig. 1. The 4-CBA DNPH with Rt of 5.2 min was characterized by Waters LCMS in electron spray negative ionization mode (ESI -ve ion mode). This peak has m/z of 329 and correlates with 4-CBA DNPH molecular weight. Its mass spectrum can be seen in Fig. 2.

### 2-4 Determination of suitable amount of DNPH

To maximize the sensitivity of the method, the volume of the derivatizing agent required for fixed quantity of sample was investigated.

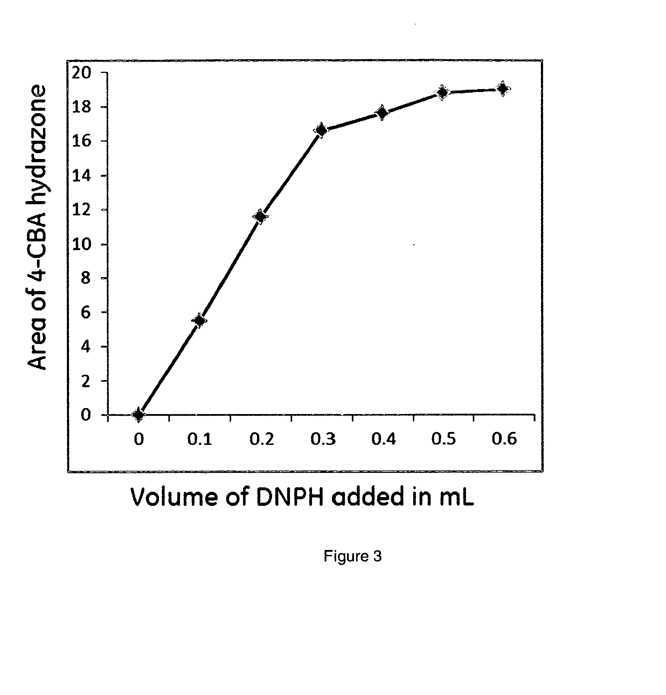

Standard and samples were derivatised as explained in section 2-1. To study the effect of the amount of DNPH required to complete the derivatization, different volumes of DNPH reagent (0.05 M) were added to 5mL of 1 ug/mL solution of standard 4-CBA, while all other parameters were held constant and injected into HPLC. Satisfactory response was obtained when 0.50 mL of 0.05M DNPH was added to fixed concentration of 4-CBA standard. It was observed that addition of larger volumes of DNPH reagent leads to precipitation of PTA and in such cases additional volume of DMSO was required to get clear solution. Fig. 3 shows the effect of DNPH volume of the response of 4-CBA hydrazone peak.

### 2-5. Precision (repeatability)

System precision of the method was evaluated by injecting the 0.05 µg/mL concentration standard of 4-CBA DNPH solution six times. %RSDs of 4-CBA hydrazone peak area and retention time were considered for calculating precision of the method. The %RSD of both the peak area and retention time (t_{R}) are listed in Table 4.

**Table 4.**

| Precision (repeatability) of 4-CBA (0.05µg/mL) injections in HPLC. | | |
|---|---|---|
| Injection (%RSD) | Area (%RSD) | t_{R} |
| n=6 | 0.68 | 0.07 |

Percent relative standard deviation (%RSD) value is within 2, indicating good precision of the method.

### 2-6 Specificity to 4-CBA DNPH

The specificity of the method was determined by injecting a blank solution prepared by the same method as sample as described in section 2-1, but without adding the PTA sample. The blank sample thus prepared, showed no interfering peaks at the retention time of the analyte. Hence, this method was found to be specific for the analysis of 4-CBA DNPH and therefore 4-CBA. Chromatogram of blank was shown in figure 1 (a).

### 2-7 LOD/LOQ

Limit of detection (LOD) and limit of quantitation (LOQ) were evaluated from signal-to-noise ratios. This was determined from the six replicate injections of solutions used for making the calibration graph (0.05 and 0.025 µg/mL). The concentration level where the S/N was approximately 10 was considered to be LOQ. Similarly, the detection limits (LOD) were based on a S/N value close to 3. The limits of quantitation (LOQ) and limit of detection (LOD) of this method for 4-CBA hydrazone was found to be 0.05 µg/mL and 0.025 µg/mL respectively.
Figure 1 shows a typical chromatogram of (a) Blank, (b) standard 4-CBA DNPH at LOQ and (c) PTA sample. Column Agilent Zorbax Eclipse C18 (150 mm X 4.6 mm X 5 mm), Mobile phase 1 : 1 (v/v) mixture of acetonitrile and 1.0% (w/v) aqueous acetic acid solution, flow rate 1.0 mL/min, detection wavelength 385 nm, temperature ambient, injection volume 25 µL, chromatographic runtime was set at 15 min.
Figure 2 shows ESI- spectrum of 4CBA hydrazone (t_{R} -5.2 min).
Figure 3 shows the effect of volume of DNPH on 4-CBA derivatisation. 4-CBA (1µg/mL) solution was treated with different volume of DNPH by keeping all other parameters constant and measured by HPLC.

## Claims

1. A method for determining the concentration of 4-carboxy benzaldehyde in a terephthalic acid composition, comprising
a) reacting 4-carboxy benzaldehyde in the terephthalic acid composition with 2,4-dinitrophenylhydrazine to obtain 4-carboxy benzaldehyde 2,4-dinitrophenylhydrazone and
b) determining the concentration of 4-carboxy benzaldehyde 2,4-dinitrophenylhydrazone.

2. The method according to claim 1, wherein step b) is performed by high performance liquid chromatography (HPLC).

3. The method according to claim 2, wherein the HPLC involves detecting the concentration of 4-carboxy benzaldehyde 2,4-dinitrophenylhydrazone by a UV detector.

4. The method according to any one of the preceding claims, wherein the TA composition is made by step-wise oxidation of p-xylene.

5. The method according to any one of the preceding claims, wherein the concentration of 4-carboxy benzaldehyde in the terephthalic acid composition is 5.8 µg/g to 357 µg/g, as determined by the method.

6. The method according to any one of the preceding claims, wherein the terephthalic acid composition is a powder composition and step a) is performed by a1) dissolving the terephthalic acid composition in dimethyl sulfoxide (DMSO) to obtain a DMSO solution and a2) adding a HCl solution of dinitrophenylhydrazone to the DMSO solution.

7. The method according to claim 6, wherein the amount of DMSO in step a1) is 10-30mL per g of the terephthalic acid composition.

8. The method according to claim 6 or 7, wherein the HCl solution of dinitrophenylhydrazone in step a2) has a concentration of 0.01-0.1 M.

9. The method of any one of the preceding claims, wherein the amount of dinitrophenylhydrazone in step a) is 0.01 to 0.1 mmol per g of the terephthalic acid composition.

10. A process for setting a condition for purifying a crude terephthalic acid composition, comprising the steps of:
- purifying the crude terephthalic acid composition at multiple conditions,
- determining for each condition the concentration of 4-carboxy benzaldehyde in the purified terephthalic acid composition by the method according to any one of the preceding claims and
- setting the condition based on the determined concentrations of 4-carboxy benzaldehyde for the respective condition.

11. A process for the preparation of a purified terephthalic acid composition, comprising the steps of: performing the process according to claim 10 and purifying a crude terephthalic acid composition according to the condition set by the process according to claim 10.
